# EUROPEAN PATENT SPECIFICATION

(11) **EP 1 115 885 B1**
(45) Date of publication and mention of the grant of the patent: **20.08.2008**
(21) Application number: 99948833.1
(22) Date of filing: 22.09.1999
(51) Int. Cl.: C12Q 1/68

(54) **IDENTIFICATION OF MICROORGANISMS CAUSING ACUTE RESPIRATORY TRACT INFECTIONS (ARI)**
IDENTIZIERUNG VON MICROORGANISMEN,DIE AKUTE ATEMWEGSINFEKTIONEN VERURSACHEN
IDENTIFICATION DES MICRO-ORGANISMES RESPONSABLES D'INFECTIONS RESPIRATOIRES

(30) Priority: 24.09.1998 EP 98870203
(43) Date of publication of application: 18.07.2001
(73) Proprietor: Innogenetics N.V., 9052 Ghent (BE)
(72) Inventor: JANNES, Geert, B-3010 Leuven (BE); SCHMITT, Heinz-Josef, D-24113 Molssee (DE)
(74) Representative: Visser-Luirink, Gesina
(86) International application number: PCT/EP1999/007065
(87) International publication number: WO 2000/017391

(56) References cited:
- EP-A- 0 576 743
- WO-A-98/11259
- DE-A- 19 716 456
- MONTO A S ET AL: "Acute respiratory infections (ARI) in children: prospects for prevention" VACCINE, vol. 16, no. 16, October 1998 (1998-10), page 1582-1588 XP004129212
- SAIKKU P: "Atypical resipratory pathogens" CLINICAL MICROBIOLOGY AND INFECTION, vol. 3, no. 6, December 1997 (1997-12), pages 599-604, XP002098063 cited in the application
- GILBERT L ET AL: "Diagnosis of viral respiratory tract infections in children by using a reverse transcription-PCR Panel" JOURNAL OF CLINICAL MICROBIOLOGY, vol. 34, no. 1, January 1996 (1996-01), pages 140-3, XP002098027 cited in the application
- TROLLFORS B ET AL: "Childhood pneumonia: possibilities for aetiological diagnosis" BAILLIERE'S CLINICAL PEDIATRICS, vol. 5, 1997, pages 71-84, XP002098033 cited in the application
- ECHEVARRIA J ET AL: "Simultaneous detection and identification of human parainfluenza viruses 1,2 and 3 from clinical samples by multiplex PCR." JOURNAL OF CLINICAL MICROBIOLOGY, vol. 36, no. 5, May 1998 (1998-05), page 1138-91 XP000876835
- EMBL Database Entry Q06252 Accession Number Q06252: 31 Jan 1991 University Patents INC."Probes for detecting picornavirus." XP002098007
- EMBL database entry T10170 Accession Number T10170, 24 July 1996 Mitsubishi Yuka BCL KK. "Adenovirus 3' primer binds to bases 2842-2861." XP002098008

## Description

The present invention relates to the field of detection of microorganisms, more particularly detection of acute respiratory tract infections.

Acute respiratory tract infections (ARIs) are the most common cause of childhood morbidity and mortality world-wide, accounting for about 30 % of all childhood deaths in the developing world (Hinman *et al.,* 1998). While rarely causing death in industrialized countries, ARIs cause enormous direct and indirect health care costs (Garenne *et al.,* 1992; UNICEF, 1993; Dixon, 1985). The causative agents of ARIs encompass a wide variety of microorganisms. *Streptococcus pneumoniae, Haemophilus influenzae* and *Moraxella caterrhalis* (Barlett and Mundy, 1995) are the most common bacteria encountered. As commensals of the upper respiratory tract the usually contaminate sputum samples, nasopharyngeal aspirates or swabs and thus their etiological role in ARIs is difficult to prove by upper respiratory tract sampling, unless invasive techniques (lung puncture) are used (Trolfors and Claesson, 1997; Nohynek *et al.,* 1995).

In contrast to these bacteria, detection of *Mycoplasma pneumoniae, Chlamydia pneumoniae* and also the detection of viruses in a child with respiratory symptoms is usually considered as evidence of acute infection. Current methods to identify these agents include cell culture, rapid antigen detection assays, serology (indirectly) and recently, PCR (Trolfors and Claesson, 1997; Saikku, 1997). Cell culture techniques require specialized laboratories, are expensive, time consuming and labour intensive. Rapid antigen assays are available for a few microorganisms only (influenza A and RSV in most countries). Serology usually requires documentation of a rise of antibody concentration from an acute to a convalescent blood sample and thus test results come in too late to be of relevance for the treatment of acute disease. While currently no rapid method for microbiological diagnosis of ARI is available for routine use, availability of such a test could result in less and more precisely tailored antibiotic therapies, resulting in reduced costs, less side effects as well as in a reduction of the emergence of resistance (Woo et al., 1997).

Currently available nucleic acid amplification techniques such as PCR (Saiki et al., 1988) and RT-PCR are highly sensitive techniques for the detection of nucleic acid sequences from viruses and bacteria in clinical specimens (Saiki, 1990; Kawasaki, 1990). These amplification techniques are particularly advantageous for detecting fastidious or "difficult to culture" organisms such as the respiratory syncytial virus (Paton *et al,* 1992) or *M. pneumoniae* (Van Kuppeveld *et al.,* 1992).

Previous studies using PCR and RT-PCR for the diagnosis of ARIs have focused on the detection of a single virus of bacterium; however, the diagnostic utility of nucleic acid amplification techniques for a single infectious agent is limited by the inability to establish a specific aetiology whenever the result is negative and by the inability to document simultaneous infections involving more than one infectious organism.

Published multiplex-PCR assays for the simultaneous detection of pathogens (Hassan-King *et a*/., 1996, 1998; Messmer *et al.,* 1997) and multiplex-RT-PCR panel to respiratory specimens as described by Gilbert et al. (1996) has the disadvantage of different and time consuming assay conditions for each detected organism and the use of several tubes for one sample, thus enlarging the risk of cross-contamination.

Our strategy to overcome these limitations was to use a multiplex -RT-PCR protocol that allows the simultaneous detection of respiratory pathogens within one working day including RNA-viruses (enteroviruses, influenza A and B viruses, parainfluenzavirus type 1 and 3, respiratory syncytial virus), a DNA-virus (adenovirus) and *bacteria* (*C. pneumoniae, M. pneumoniae*) that do not usually colonize the upper respiratory tract of children.

The aim of the present invention is to provide methods and kits for detecting acute respiratory tract infections.

More particularly it is an aim of the present invention to provide a multiplex PCR method and kit for detecting acute respiratory tract infections.

It is also an aim of the present invention to provide mixtures of primers and mixtures of probes useful for detecting acute respiratory tract infections.

More particularly the present invention relates to a method for the detection of acute respiratory tract infection comprising the simultaneous amplification of several target nucleotide sequences present in a biological sample by means of a primer mixture comprising at least one primer set from each one of the following gene regions:
- the F1 subunit of the fusion glycoprotein gene for RSV,
- the hemagglutininneuraminidase gene for PIV-1,
- the 5' noncoding region of the PIV-3 fusion protein gene,
- 16 S rRNA sequence for *M. pneumoniae,*
- 16S rRNA sequence for *C*. *pneumoniae,*
- the 5' noncoding region for enterovirus,
- the non-structural protein gene from influenza A,
- the non-structural protein gene from influenza B, and,
- the hexon gene for adenoviruses.

This multiplex RT-PCR method is particularly preferred because it allows to determine the presence of the following micro organisms which infect the respiratory tract of mainly children in one amplification step: RSV, parainfluenza virus, *M. pneumoniae, C. pneumoniae,* enterovirus, influenza A and B and adenoviruses.

According to a preferred embodiment, the present invention relates to a method as defined above wherein said 16S rRNA primers are replaced by primers from the spacer region between the 16S and the 23S rRNA sequences.

According to another embodiment, the present invention relates to a method as defined above wherein in addition also at least one primer pair for the specific detection of *B. pertussis* and *B. parapertussis* are used, with said primers being preferably from the spacer region between the 16S and 23S rRNA sequences.

According to another embodiment, the present invention relates to a method as defined above wherein said primers are chosen from Table 2 or Table 4.

According to another embodiment, the present invention relates to a method as defined above wherein said amplified products are subsequently detected using a probe, with said probe being preferably selected from Table 3, Table 4 or Table 5.

According to another embodiment, the present invention relates to a primer mixture containing at least nine primer sets chosen from the group consisting of SEQ ID NOs 17, 18, 19, 20, 21, 22, 23 and primer sequences of Table 2, whereby the mixture contains at least one primer set front each one of the gene region listed above.

According to another embodiment, the present invention relates to a probe mixture containing at least nine probes chosen from SEQ ID NOs 5,6,7,8, 9,10,11,12,13,14,13, 16, 24,25, 26, 27, 28, 29; 30, 31, 32,33 and 34 and their complementary form and their RNA form wherein T is replaced by U, whereby the mixture contains for each one of the gene regions listed above at least one specific probe, for the simultaneous detection of respiratory pathogens.

According to another embodiment, the present invention relates to a kit for the detection of acute respiratory tract infection comprising a mixture of primers as defined above for performing a method as defined above. Besides said primers, such a kit may also contain probes as well as the necessary buffers for achieving the amplification and possible hybridization reactions as well as a kit insert.

According to another embodiment, the present invention relates to a kit for the detection of acute respiratory tract infection comprising a mixture of probes for performing a method as defined above. Besides said probes, such a kit may also contain primers as well as the necessary buffers for achieving the hybridization and possible amplification reactions as well as a kit insert.

According to another embodiment, the present invention relates to a kit as defined above, wherein said probes are applied as parallel lines on a solid support, preferably on a nylon membrane, preferably a LiPA kit (see examples section and below).

Different techniques can be applied to perform the methods of the present invention. These techniques may comprise immobilizing the target polynucleic acids, after amplification, on a solid support and performing hybridization with labelled oligonucleotide probes. Alternatively, the probes may be immobilized on a solid support and hybrdization may be performed with labelled target polynucleic acids, possibly after amplification. This technique is called reverse hybridization. A convenient reverse hybridization technique is the line probe assay (LiPA, Innogenetics, Belgium). This assay uses oligonucleotide probes immobilized as parallel lines on a solid support strip. Alternatively the probes may be present on an array or micro-array format. The probes can be spotted onto this array or synthesized in situ on the array (Lockhart *et al.,* 1996) in discrete locations. It is to be understood that any other technique for detection of the above-mentioned co-amplified target sequences also covered by the present invention. Such a technique can involve sequencing or other array methods known in the art.

The following definitions and explanations will permit a better understanding of the present invention.

The target material in the samples to be analysed may either be DNA or RNA, e.g. genomic DNA, messenger RNA or amplified versions thereof. These molecules are in this application also termed "polynucleic acids".

Well-known extraction and purification procedures are available for the isolation of RNA or DNA from a sample (e.g. in Sambrook *et al.,* 1989).

The term "probe" according to the present invention refers to a single-stranded oligonucleotide which is designed to specifically hybridize to the target polynucleic acids. Preferably, the probes of the invention are about 5 to 50 nucleotides long, more preferably from about 10 to 25 nucleotides. Particularly preferred lengths of probes include 10, 11, 12, 13, 14, 15, 16, 17, 18, 19, 20, 21, 22, 23, 24 or 25 nucleotides. The nucleotides as used in the present invention may be ribonucleotides, deoxyribonucleotides and modified nucleotides such as inosine or nucleotides containing modified groups which do not essentially alter their hybridization characteristics.

The term "primer" refers to a single stranded oligonucleotide sequence capable of acting as a point of initiation for synthesis of a primer extension product which is complementary to the nucleic acid strand to be copied. The length and the sequence of the primer must be such that they allow to prime the synthesis of the extension products. Preferably the primer is about 5-50 nucleotides long. Specific length and sequence will depend on the complexity of the required DNA or RNA targets, as well as on the conditions at which the primer is used, such as temperature and ionic strength. It is to be understood that the primers of the present invention may be used as probes and *vice versa,* provided that the experimental conditions are adapted.

The expression "suitable primer pair" in this invention refers to a pair of primers allowing specific amplification of a specific target polynucleic acid fragment.

The term "target region" of a probe or a primer according to the present invention is a sequence within the polynucleic acids to be detected to which the probe or the primer is completely complementary or partially complementary (i.e. with some degree of mismatch). It is to be understood that the complement of said target sequence is also a suitable target sequence in some cases.

"Specific hybridization" of a probe to a target region of a polynucleic acid means that said probe forms a duplex with part of this region or with the entire region under the experimental conditions used, and that under those conditions said probe does not form a duplex with other regions of the polynucleic acids present in the sample to be analysed.

"Specific hybridization" of a primer to a target region of a polynucleic acid means that, during the amplification step, said primer forms a duplex with part of this region or with the entire region under the experimental conditions used, and that under those conditions said primer does not form a duplex with other regions of the polynucleic acids present in the sample to be analysed. It is to be understood that "duplex" as used hereby, means a duplex that will lead to specific amplification.

The fact that amplification primers do not have to match exactly with the corresponding target sequence in the template to warrant proper amplification is amply documented in the literature (Kwok *et al.,* 1990). However, when the primers are not completely complementary to their target sequence, it should be taken into account that the amplified fragments will have the sequence of the primers and not of the target sequence. Primers may be labelled with a label of choice (e.g. biotin). The amplification method used can be either polymerase chain reaction (PCR; Saiki *et al.,* 1988), ligase chain reaction (LCR; Landgren *et al.,* 1988; Wu & Wallace, 1989; Barany, 1991), nucleic acid sequence-based amplification (NASBA; Guatelli *et al.,* 1990; Compton, 1991), transcription-based amplification system (TAS; Kwoh *et al.,* 1989), strand displacement amplification (SDA; Duck, 1990) or amplification by means of QB replicase (Lomeli *et al.,* 1989) or any other suitable method to amplify nucleic acid molecules known in the art.

Probe and primer sequences are represented throughout the specification as single stranded DNA oligonucleotides from the 5' to the 3' end. It is obvious to the man skilled in the art that any of the below-specified probes can be used as such, or in their complementary form, or in their RNA form (wherein T is replaced by U).

The probes according to the invention can be prepared by cloning of recombinant plasmids containing inserts including the corresponding nucleotide sequences, if need be by excision of the latter from the cloned plasmids by use of the adequate nucleases and recovering them, e.g. by fractionation according to molecular weight. The probes according to the present invention can also be synthesized chemically, for instance by the conventional phospho-triester method.

The oligonucleotides used as primers or probes may also comprise nucleotide analogues such as phosphorothiates (Matsukura *et al*., 1987), alkylphosphorothiates (Miller *et al.,* 1979) or peptide nucleic acids (Nielsen *et al.,* 1991, 1993) or may contain intercalating agents (Asseline *et* a/., 1984). As most other variations or modifications introduced into the original DNA sequences of the invention these variations will necessitate adaptations with respect to the conditions under which the oligonucleotide should be used to obtain the required specificity and sensitivity. However the eventual results of hybridization will be essentially the same as those obtained with the unmodified oligonucleotides. The introduction of these modifications may be advantageous in order to positively influence characteristics such as hybridization kinetics, reversibility of the hybrid-formation, biological stability of the oligonucleotide molecules, etc.

The term "solid support" can refer to any substrate to which an oligonucleotide probe can be coupled, provided that it retains its hybridization characteristics and provided that the background level of hybridization remains low. Usually the solid substrate will be a microtiter plate, a membrane (e.g. nylon or nitrocellulose) or a microsphere (bead) or a chip. Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin, haptens or proteins.

The term "labelled" refers to the use of labelled nucleic acids. Labelling may be carried out by the use of labelled nucleotides incorporated during the polymerase step of the amplification such as illustrated by Saiki *et al.* (1988) or Bej *et al.* (1990) or labelled primers, or by any other method known to the person skilled in the art. The nature of the label may be isotopic (³²P, ³⁵S, etc.) or non-isotopic (biotin, digoxigenin, etc.).

The term "biological sample or sample" refers to for instance naso-pharyngeal aspirates, throat or nasopharyngeal swabs, nasopharyngeal washes or tracheal aspirates or other respiratory tract sample comprising DNA or RNA.

For designing probes with desired characteristics, the following useful guidelines known to the person skilled in the art can be applied.

Because the extent and specificity of hybridization reactions such as those described herein are affected by a number of factors, manipulation of one or more of those factors will determine the exact sensitivity and specificity of a particular probe, whether perfectly complementary to its target or not. The importance and effect of various assay conditions are explained further herein.

The stability of the [probe : target] nucleic acid hybrid should be chosen to be compatible with the assay conditions. This may be accomplished by avoiding long AT-rich sequences, by terminating the hybrids with G:C base pairs, and by designing the probe with an appropriate Tm. The beginning and end points of the probe should be chosen so that the length and %GC result in a Tm about 2-10°C higher than the temperature at which the final assay will be performed. The base composition of the probe is significant because G-C base pairs exhibit greater thermal stability as compared to A-T base pairs due to additional hydrogen bonding. Thus, hybridization involving complementary nucleic acids of higher G-C content will be more stable at higher temperatures.

Conditions such as ionic strength and incubation temperature under which a probe will be used should also be taken into account when designing a probe. It is known that the degree of hybridization will increase as the ionic strength of the reaction mixture increases, and that the thermal stability of the hybrids will increase with increasing ionic strength. On the other hand, chemical reagents, such as formamide, urea, DMSO and alcohols, which disrupt hydrogen bonds, will increase the stringency of hybridization. Destabilization of the hydrogen bonds by such reagents can greatly reduce the Tm. In general, optimal hybridization for synthetic oligonucleotide probes of about 10-50 bases in length occurs approximately 5°C below the melting temperature for a given duplex. Incubation at temperatures below the optimum may allow mismatched base sequences to hybridize and can therefore result in reduced specificity.

It is desirable to have probes which hybridize only under conditions of high stringency. Under high stringency conditions only highly complementary nucleic acid hybrids will form; hybrids without a sufficient degree of complementarity will not form. Accordingly, the stringency of the assay conditions determines the amount of complementarity needed between two nucleic acid strands forming a hybrid. The degree of stringency is chosen such as to maximize the difference in stability between the hybrid formed with the target and the non-target nucleic acid.

Regions in the target DNA or RNA which are known to form strong internal structures inhibitory to hybridization are less preferred. Likewise, probes with extensive self-complementarity should be avoided. As explained above, hybridization is the association of two single strands of complementary nucleic acids to form a hydrogen bonded double strand. It is implicit that if one of the two strands is wholly or partially involved in a hybrid that it will be less able to participate in formation of a new hybrid. There can be intramolecular and intermolecular hybrids formed within the molecules of one type of probe if there is sufficient self complementarity. Such structures can be avoided through careful probe design. By designing a probe so that a substantial portion of the sequence of interest is single stranded, the rate and extent of hybridization may be greatly increased. Computer programs are available to search for this type of interaction. However, in certain instances, it may not be possible to avoid this type of interaction.

Standard hybridization and wash conditions are disclosed in the Materials & Methods section of the Examples. Other conditions are for instance 3X SSC (Sodium Saline Citrate), 20% deionized FA (Formamide) at 50°C. Other solutions (SSPE (Sodium saline phosphate EDTA), TMAC (Tetramethyl ammonium Chloride), etc.) and temperatures can also be used provided that the specificity and sensitivity of the probes is maintained. When needed, slight modifications of the probes in length or in sequence have to be carried out to maintain the specificity and sensitivity required under the given circumstances.

The term "hybridization buffer" means a buffer allowing a hybridization reaction between the probes and the polynucleic acids present in the sample, or the amplified products, under the appropriate stringency conditions.

The term "wash solution" means a solution enabling washing of the hybrids formed under the appropriate stringency conditions.

The invention, now being generally described, will be more readily understood by reference to the following examples and figures, which are included merely for the purposes of illustration of certain aspects and embodiments of the present invention and are in no way to be construed as limiting the present invention.

### BRIEF DESCRIPTION OF THE FIGURES AND TABLES

**Figure 1****.** Separation of m-RT-PCR products on agarose Gel. 10 µl of m-RT-PCR products were separated on 2 % agarose gel. The m-RT-PCR was performed using 1 µl of viral or bacterial nucleic acid as template as described in material and methods. The expected product lengths are given in the text. DNA fragment size of marker (0.7 µg of Mspl digested pUC19) in base pairs (bp) is 1: 501 bp; 2: 404 bp; 3: 331 bp; 4: 242 bp; 5: 190 bp; 6: 147 bp; 7: 110 bp.
**Figure 2****.** Proportion of positive m-RT-PCR results. The number of positive m-RT-PCR results and total samples is given on the y-axis. The time scale on the x-axis is from November 1995 to April 1998.
**Figure 3****.** Frequency of positive m-RT-PCR-ELISA results in clinical specimens. The number of positive m-RT-PCR results for each of the nine organisms is given on the y-axis. The time scale on the x-axis is from November 1995 to April 1998.
**Figure 4****.** Percentage of organisms causing infections. The amount of organisms causing a respiratory disease is given in percent of the total of organisms causing the according disease. Organisms not included in the test are not shown in the figure.
**Figure 5** shows the separation on a 2% agarosegel of the amplicons obtained after multiplex-RT-PCR on reference material shows discrete bands of the expected size for all organisms tested.
**Figure 6** shows hybridization results of these amplicons with the LiPA strips as well as a negative control. These results clearly demonstrate that all the probes on the strip react specifically with their corresponding amplicons and no cross-hybridization is seen between the different organisms tested.

**Table 1** shows the results from a comparative study between m-RT-PCR-ELISA and commercial EIA's.
**Table 2** shows the primer sequences used in Example 1.
**Table 3** summarizes the different probes for the organisms identified as originally described and their adapted versions for LiPA use.
**Table 4** summarizes the sequences of the primers and probes, derived from the 16S-23S rRNA spacer region for the bacterial pathogens.
**Table 5** shows the sequences of probes for RSV used in Example 3.
**Table 6** shows a comparison of culture and LiPA results for a series of 36 blinded samples, performed in Example 3.
**Table 7.** shows a comparison of culture and LiPA results for a series of 30 blinded specimens for culture of *Mycoplasma pneumoniae* using multiplex-RT-PCR and LiPA, performed in Example 3.

### EXAMPLES

### Example 1

### Abbreviations.

Acute respiratory tract infection (ARI); reverse transcription combined with PCR (RT-PCR); multiplex-RT-PCR (m-RT-PCR); m-RT-PCR combined with microwell hybridization assay (m-RT-PCR-ELISA); influenzavirus type A (influenza A or InfA); influenzavirus type B (influenza B or InfB); parainfluenzavirus type 1 (PIV-1); parainfluenzavirus type 3 (PIV-3); respiratory syncytial virus (RSV).

### MATERIALS AND METHODS

### 1. Patient samples

Nasopharyngeal aspirates were obtained from children hospitalized with ARI at our institution in the time between November 1995 through April 1998. Diagnosis included pneumonia, wheezing bronchitis, bronchitis, laryngotracheitis (the latter encompassing laryngitis, laryngo-tracheo-bronchitis and (pseudo-) croup), pharyngitis, tonsillitis, rhinitis, conjunctivitis, otitis media and were obtained from the computer-based discharge-diagnosis database of the hospital. While specimen collection was not complete during the first winter season (November 1995 to April 1996), it was >95 % complete for the remaining time as October 1 st, 1996. Specimens were collected the first working day following hospitalization, directly brought to the laboratory, initially stored at 4°C, prepared for testing and afterwards or for longer storage frozen at minus 70°C. Samples were split with appropriate precautions to avoid contamination and one portion was used directly for detection of RSV and influenza type A antigen by the use of enzyme immuno assays (EIA) (Becton Dickinson, Heidelberg, Germany). A second portion was used for m-RT-PCR followed by agarose gel electrophoresis and specification in a microwell hybridization assay.

### 2. Nucleic acid extraction

Samples received from November 1995 through July 1997 were prepared as follows. Total nucleic acids were obtained from 100 µl of respiratory specimens diluted with 100 µl 0,9 % NaCl-solution. Sodiumdodecyl sulphate was added to a final concentration of 0,1 %. Nucleic acid extraction was accomplished once with 1 volume of 1:1 phenol-chloroform mixture and once with 1 volume chloroform and precipitated with 0,3 M ammonium acetate and ethanol. Nucleic acid pellets were dried and resuspended in 15 µl of diethylpyrocarbonate-treated, bidistilled water. Specimens received from August 1997 to April 1998 were prepared using the Boehringer "High Pure Viral Nucleic Acid Kit" following the instructions of the purchaser (Boehringer Mannheim, Mannheim, Germany).

Controls of the preparation procedure were as follows: One negative sample (sputa from healthy persons) was included in each series of 5-10 samples to monitor for potential cross-contamination. In case of a false positive result in the negative control, the m-RT-PCR was repeated on all positive clinical samples in that series with another portion of the clinical specimen. Positive controls from culture (influenza A, influenza B, PIV-1, PIV-3 or RSV respectively) were used in each test to document the efficiency of the preparation procedure. Prepared samples were used immediately for m-RT-PCR and remaining aliquots were stored at minus 70°C.

### 3. Multiplex-RT-PCR

Target sequences were coding/non-coding regions, respectively, of: F1 subunit of the fusion glycoprotein gene for RSV, hemagglutininneuraminidase gene for PIV-1,5' noncoding region of the PIV-3 fusion protein gene, nucleotide sequence of the 16S ribosomal RNA for *M*. *pneumoniae,* nucleotide sequence of the 16S ribosomal RNA for *C*. *pneumoniae,* nucleotide sequence of the 16S ribosomal RNA for *C. pneumoniae,* an among enteroviruses highly conserved 5' noncoding region for enterovirus, non-structural protein gene from influenza A and influenza B and sequence of the hexon gene for adenoviruses. Sequences were selected from procedures described previously (Paton *et al.,* 1992; Karron *et al.,* 1992; Fan and Henrickson, 1996; Rotbart, 1990; Gaydos *et al.,* 1992; Van Kuppeveld *et al.,* 1992; Claas *et al,1992;* Hierholzer *et al* 1993). For adenovirus the sequence of probe A was used as second amplification primer instead of primer 2 (Hierholzer, 1993).

Five to six µl of the nucleic preparations from clinical specimens were included in the reverse transcription (RT) reaction in a final volume of 20 µl. The RT was performed for 60 min at 37°C with the following buffer composition: 50 mM Tris-HCl (pH 8.3), 75mM MgCl2, 10 mM (each) deoxynucleoside triphosphates (Pharmacia Biotech, Uppsala, Sweden), 0.2 µg/ul hexanucleotide mix (Boehringer Mannheim, Mannheim, Germany), 20 U RNAsin (Promega, Madison, Wisconsin USA) and 10 U of Moloney murine leukemia virus reverse transcriptase (Eurogentec, Seraing, Belgium).

After heat inactivation of reverse transcriptase at 90°C for 5 min, the entire 20-µl RT reaction mixture was used for multiplex PCR in a total volume of 80 µl. The buffer composition (without consideration of the RT-buffer) was 10 mM Tris-HCl (pH 8.3), 50 mM KCl, 1,5 mM MgCl2, 0.001 % gelatin, 0.2 mM dATP, dCTP, dGTP, 0.2 mM dTTP, 0.01 mM digoxigenin-11-dUTP (Boehringer Mannheim, Mannheim, Germany), 1 µM (each) primer (Eurogentec, Seraing, Belgium), and 5 U of AmpliTaq-Gold polymerase (Perkin-Elmer, Branchburg, NJ, USA). PCR was performed on a PE 9600 Thermocycler (Perkin, Elmer, Branchburg, NJ, USA) as follows: 40 cycles of denaturation at 94°C for 30 sec (10 min during cycle 1), annealing at 50°C for 30 sec and extension at 72°C for 30 sec (7 min during cycle 40).

As negative control bank reagent that contained H₂O was used instead of nucleic acid. As positive m-RT-PCR control total cellular nucleic acid extracted from virus and/or bacterial stocks was used.

### 4. Prevention of carry-over contamination

To prevent carry-over contamination within the laboratory, the following precautions were taken: All purchased reagents were split into small aliquots. The preparation of PCR reagents, the extraction of nucleic acids from clinical samples and the amplification step were conducted in three different rooms. Tips equipped with sealing filters (safeseal-tip from BlOzym, Hess. Oldendorf, Germany) were used for pipetting reagents introduced into the PCR and all areas and equipment were decontaminated with sodiumhypochlorite and Bacillol (an alcoholic disinfectant from: Bode Chemie, Hamburg, Germany) prior to and after pipetting.

### 5. Agarose gel electrophoresis

Electrophoretic separation of PCR products (10 µl) was performed for 30 minutes at 130 tot 160 mA on 2 % agarose gels in 0.5 x TBE buffer (0.045M Tris-borate, 0.001 M EDTA), stained with ethidium bromide and PCR products were visualized by UV illumination as described by Sambrook et al. (Sambrook *et al.,* 1989). For control of fragment lengths, 0.6 - 0.8 µg of *Msp*l digested pUC19 DNA was applied as marker.

### 6. Microwell hybridization analysis

This assay was performed using the PCR-ELISA system from Boehringer Mannheim (Mannheim, Germany). Nine wells of a streptavidin-coated microtiter plate were each filed with 5 µl of PCR product and denatured by adding 25 µl of 0.2 N NaOH to each well. After 5 minutes 200 µl hybridization buffer containing 2 pmol of the respective biotinylated capture probe was added. The capture probes used were specific for the amplified target sequences (see above) and the sequences of the probes for enterovirus, influenza A, influenza B, PIV-1, adenovirus (probeB) and *M. pneumoniae* (Gpo1) were identical to previously reported sequences (Rotbart, 1990; Claas et al., 1992; Van Kuppenveld et al., 1992; Hierholzer et al., 1993; Fan and Hendricksom, 1996). The sequences of probes used for the others are 5'-CCT GCA TTA ACA CTA AAT TC-3' (SEQ ID NO 1) for RSV; 5'-TCT TGC TAC CTT CTG TAC TAA-3' (SEQ ID NO 2) for *C*. *pneumonia* and 5'-AAA ATT CCA AAA GAG ACC GGC-3' (SEQ ID NO 4) for PIV-3. All capture probes were 3'-biotinylated and purchased by Eurogentec (Seraing, Belgium), Capture was allowed to proceed for 1 h at 37°C, and afterwards the wells were washed four times with 200 µl of washing solution (Boehringer Mannheim, Mannheim, Germany) at room temperature. To each well 200 µl of anti-DIG-peroxidase (10 mU/ml, Boehringer Mannheim, Mannheim, Germany) diluted 1/1,000 in a buffer containing 100 mM Tris-HCl, 150 mM NaCl (pH 7.5) was added. Plates were incubated for 30 min at 37°C and wells were washed four times with washing solution. 200 µl ABTS^{®} substrate solution (Boehringer Mannheim, Mannheim, Germany) was added, and the wells were incubated for 30 min at 37°C. The optical density (OD) was read on a DIAS reader (Dynatech Laboratories, Guernsey, Channel Islands) at 405 nm (reference filter 492 nm). The run was considered valid, if all negative control values were less than 0.2 OD units and the positive control was higher than 1.0 OD units. Samples were classified as PCR positive or negative according to a cut-off OD value of 0.5 and by comparison with the results from gel electrophoresis. Samples with initial readings of between 0.2 and 0.5 were considered borderline and were classified as positive or negative only after retesting with the specific single primer set. Positive hybridization controls were included in each microwell hybridization assay. They consisted of PCR products derived from the positive controls that were included in the m-RT-PCR.

### 7. Administration of data

All data obtained were managed in a Microsoft Access database. This database included all available information about patients as well as all diagnostic data and results from m-RT-PCR-ELISA, and in case of influenza A and RSV the data of the EIA.

### 8. Bacterial and viral stocks

Bacterial and viral stocks used as positive control were kindly provided by the following persons: B. Schweiger and E. Schreier, (Robert-Koch-Institute, Berlin) enteroviruses, Influenza A and influenza B; K.M. Edwards (Vanderbilt University, Tennessee, USA) RSV, PIV-1, PIV-3; A. Strecker (Institute for Virology, Bochum) RSV-long, PIV-3; R. Krausse and P. Rautenberg (Institute for Medical Microbiology, Kiel), *M*. *pneumoniae, C. pneumoniae,* and adenoviruses.

The exact number of viruses or bacteria within these samples was not known and was assumed to be at most 10⁸/ml as stated by B. Schweiger and P. Rautenberg (personal communication). For preliminary sensitivity testing of m-RT-PCR consecutive dilutions (tenfold steps) of prepared nucleic acids from these cultures were used as template for m-RT-PCR and for amplification with single primer sets.

Due to unavailable information about the exact number of viruses and bacteria the probable amount of nucleic acids which was sufficient to result in an amplification product was calculated based on the assumed particle number (10⁸/ml undiluted sample). To detect possible cross reactivity among the organisms one µl of undiluted nucleic acid from each organism was used in an m-RT-PCR.

### RESULTS

### 1. Multiplex RT-PCR on bacterial and viral nucleic acids.

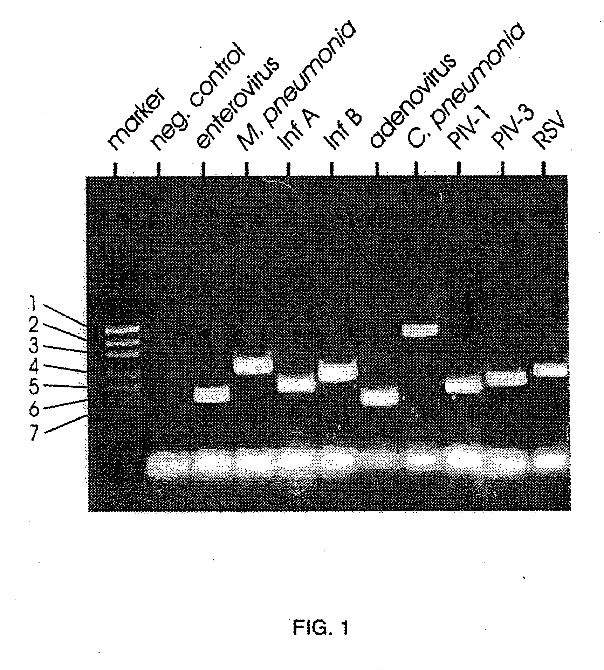

The m-RT-PCR-ELISA procedure was tested with nucleic acids prepared from the stock solutions as described in material and methods. As can be seen in figure 1 only one specific amplification product could be observed in each lane. The predicted sizes of the amplification products (*C. pneumoniae,* 463 bp; *M. pneumoniae,* 277 bp; influenza B, 249 bp; RSV, 239 bp; PIV-3, 205 bp; influenza A, 190 bp; PIV-1, 179 bp; enterovirus 154 bp; adenovirus, 134 bp) were in good agreement with the fragment sizes calculated from the agarose gel (Fig.1). This suggests that the m-RT-PCR yielded specific products. However, differentiation of influenza A and PIV-3 by fragment size in gel electrophoresis alone is difficult, but the absorbance values obtained by the PCR-ELISA test confirmed this specificity. Unconsumed primers are visible at the bottom of the gel.

In order to estimate the sensitivity of the m-RT-PCR concentrated virus stock solutions were diluted consecutively in 10-fold steps and tested with specific primer pairs to produce amplification products visible on agarose gel which were specified in the PCR-ELISA. Assuming that a maximum of 10⁸ of the respective microorganisms per ml were present in the original stock solution, it was calculated that the method was able to detect 1 target sequence of *M. pneumoniae* and 1 target sequence of *C. pneumoniae,* 10 copies of adenovirus DNA and enterovirus RNA, 100 copies of PIV-1, PIV-3, influenza A, influenza B and RSV-RNA in the analogue m-RT-PCR reaction.

### 2. Comparison of Enzyme Immuno Assay (EIA) with m-RT-PCR-ELISA.

To receive information about the quality of the m-RT-PCR-ELISA we compared results obtained with those from commercial EIA's. With this EIA 940 clinical specimens were tested for the presence of influenza A and 1.031 clinical specimens for the presence of RSV. Results are summarized in table 1. The overall accordance was 95 % of PCR results for RSV to those obtained by EIA (with 140 positive+891 negative specimens in EIA as reference = 100 %). 25 specimens were identified as RSV positive by PCR-ELISA only, 24 as RSV positive by EIA only. In case of influenza A the overall accordance of PCR to EIA was 98 % (with 53 positive + 887 negative in EIA as reference = 100 %) with 1 specimen positive by EIA only and 14 specimens positive by PCR ELISA only.

### 3. Results of m-RT-PCR-ELISA with clinical specimens.

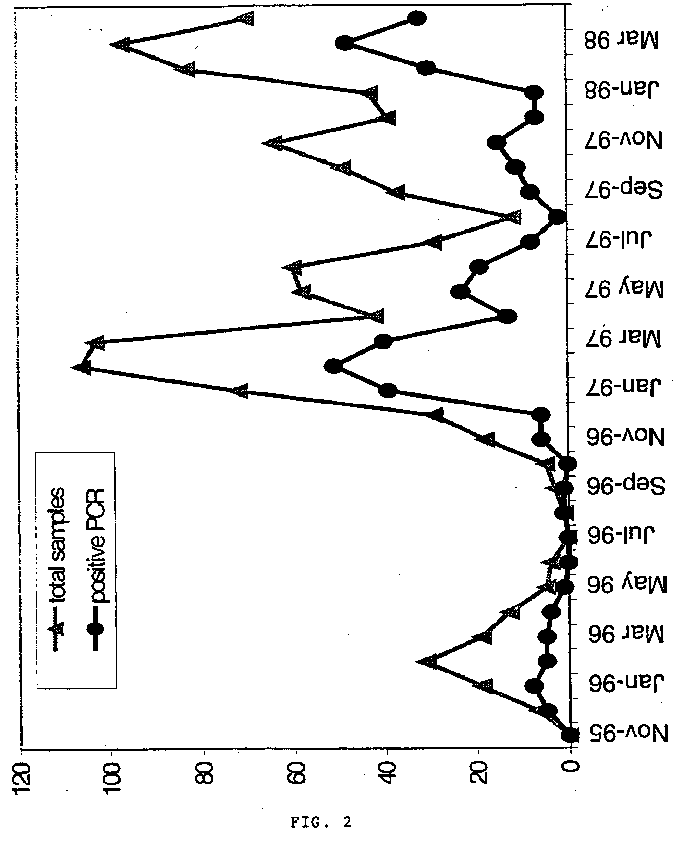

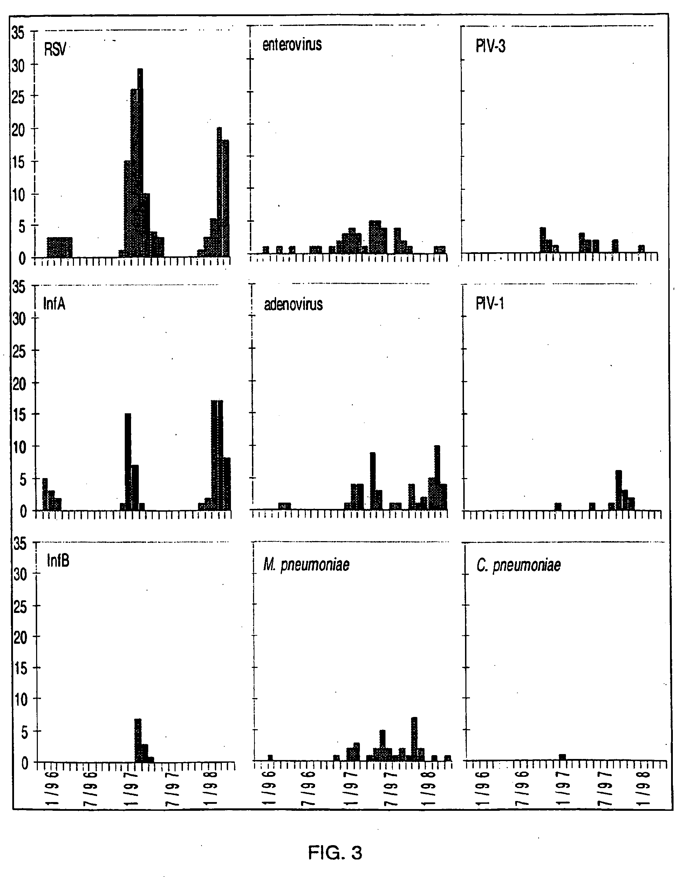

A total of 1.118 samples were tested by m-RT-PCR-ELISA. The number of samples tested over time and the proportion of positive PCR results can be taken from figure 2. The amount of specimens increased periodically during all cold seasons (from November to April) and this correlated with an increased number of positive PCR results. During the winter season 1996/1997 the maximum number of patients samples (n=106) was received in February and detection of at least one microorganism by m-RT-PCR was accomplished in 48 %. The lower number of specimens in the winter 1995/1996 was due to incomplete sample collection early during our test series. Results for the different microorganisms are shown in detail in figure 3. A total of 723 (65%) specimens were negative and 395 (35 %) were positive for at least one of the organisms included in the test. Of the isolates 37.5 % were RSV, 20.0 % influenza A, 12.9 % adenoviruses, 10.6 % enteroviruses, 8.1% *M*. *pneumoniae,* 4.3 % PIV-3, 3.5 % PIV-1, 2.8 % influenza B and 0.2% *C. pneumoniae,* (based on total positive m-RT-PCR-ELISA). RSV and influenza A mainly were detected from December to May. For influenza B (February to April 1997) and for PIV-1 (September to December 1997) only one main peak was observed. Infection with adenovirus, enterovirus, PIV-3 and *M. pneumoniae* was detected more or less constantly over the time. *C. pneumoniae* was detected only once in January 1997.

### 4. Simultaneous detection of two organisms.

The m-RT-PCR revealed evidence of simultaneous infection with two organisms in 20 cases (1.8 % of the total or 5% of the positive specimens). Co-amplification of adenovirus nucleic acid sequence occurred with *C. pneumoniae* (1x), enterovirus (1x), influenza A (1 x) and RSV (5x). Dual infections involving enteroviruses were detected with adenovirus (1 x), influenza A (3x), influenza B (1 x), PIV-3 (3x), *M. pneumoniae* (1 x) and with RSV (3x).

Furthermore influenza B nucleic acid was co-amplified with RSV and *M. pneumoniae* with PIV-1 each in one specimen.

### 5. Clinical data.

Clinical data were available as of February 1995 from 861/1.061 sample -patient pairs with second or following samples from the same hospital admission of one patient being excluded. From these 861 patients, 550 were between 0 to 2 years of age, 153 were between 2 to 5 years of age and 158 were older than 5 years. In 62 % of those specimens no bacterial or viral nucleic acids could be detected by m-RT-PCR. The most frequent diagnosis in this hospital-based study was pneumoniae (309 cases or 36 %). It was most commonly caused by RSV (n=59), influenza A (n=17), *M. pneumoniae* (n=16) and adenovirus (n=15). Enterovirus, PIV-3, PIV-1 and influenza B were associated with less than 10 pneumonia cases each. Among 167 patients with wheezing bronchitis (19 % of 861 specimens) RSV was detected in 45 cases, adenovirus, in 16 and enterovirus, influenza A, influenza B, PIV-1, PIV-3 and *M. pneumoniae* in less than 10 cases each. Bronchitis was observed in a total of 95 patients (11% of the 861 specimens) and the detected organisms were RSV (13 cases), enterovirus and influenza A (4 cases each), adenovirus (3 cases). Rhinitis was diagnosed in 7.1 %, laryngotracheitis in 6.2 %, and pharyngitis, otitis media, tonsillitis and conjunctivitis in less than 5 % each of the 861 specimens tested with the detection of a microorganism by m-RT-PCR in less than 10 cases. Other diseases were diagnosed in 9.1 % of the patients.

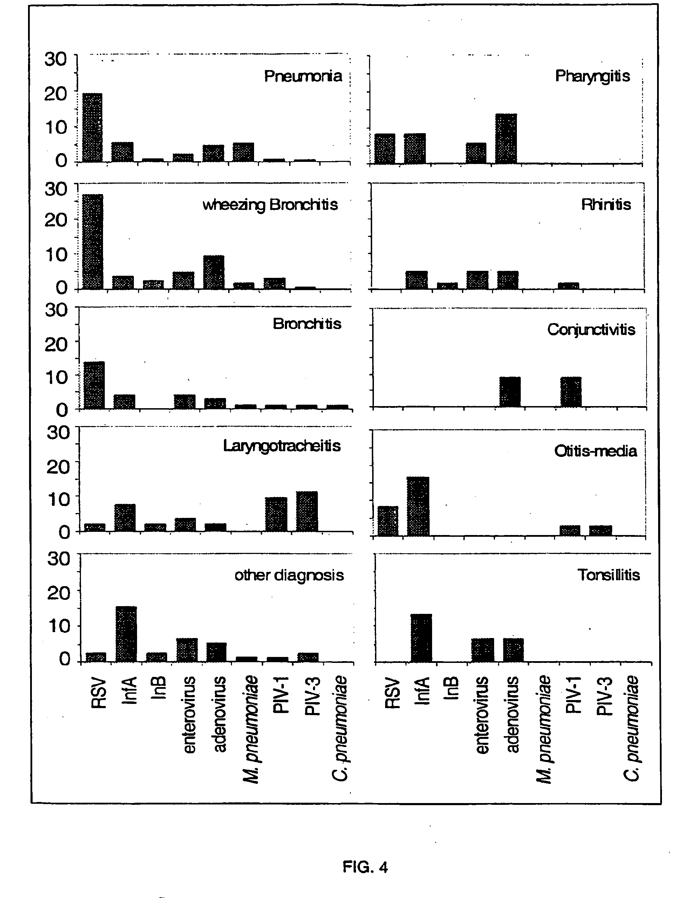

The frequency of detection of one of the nine organisms in the PCR for a given respiratory disease is shown in figure 4. RSV was most commonly associated with pneumonia, wheezing bronchitis, bronchitis, otitis media or pharyngitis. Influenza A was associated with more than 5 % of the cases of otitis media, tonsillitis, pharyngitis, laryngotracheitis, pneumonia; enteroviruses were associated with more then 5 % of cases of tonsillitis, pharyngitis; adenoviruses were associated with pharyngitis, wheezing bronchitis, conjunctivitis and tonsillitis; *M. pneumoniae* most commonly associated with pneumoniae, while PIV-1 was mainly associated with laryngotracheitis and PIV-3 with laryngotracheitis and conjunctivitis. *C. pneumoniae* was detected only once in a patient with bronchitis.

### Example 2 : LIPA application for acute respiratory tract infections

### 1. Design of oligonucleotide probes for LiPA application

Some of the oligonucleotide probes used in Example 1 were adapted in order to obtain good specificities and sensitivities for the different organisms when used in a LiPA assay (Line Probe Assay, WO 94/12670). Table 3 summarizes the different probes for the organisms identified as originally described and their adapted versions for LiPA use.

Optimized probes were provided enzymatically with a poly-T-tail using terminal deoxynucleotidyl transferase (Pharmacia) in a standard reaction buffer. After one hour incubation, the reaction was stopped and the tailed probes were precipitated and washed with ice-cold ethanol. Probes were dissolved in 6x SSC at their respectively specific concentrations and applied as horizontal lines on membrane strips. Biotinylated DNA was applied alongside as positive control. The oligonucleotides were fixed to the membrane by baking at 80°C for 12 hours. The membrane was than sliced into 4mm strips.

### 2. Nucleic acid preparation and PCR amplification

Bacterial and viral culture stocks were used as reference material. Nucleic acid was extracted according to standard procedures as described above.

One to five µl of the nucleic acid preparation was used in the multiplex-RT-PCR as described previously, with the exception that the cycle number was reduced to 35 and labelling of the amplicons was done by using biotinylated primers instead of the incorporation of digoxigenin-11-dUTP.

### 3. LiPA test performance

Equal volumes (5 to 10 µl) of the biotinylated PCR fragments and of the denaturation solution (400 mM NaOH/10 mM EDTA) were mixed in test troughs and incubated at room temperature for 5 min. Then, 2 ml of the 50°C prewarmed hybridization solution (2xSSC/0.1 % SDS) was added followed by the addition of one strip per test trough. Hybridization occurred for 1 hour at 50°C in a closed shaking water bath. The strips were washed twice with 2 ml of stringent wash solution (2xSSC/0.1 % SDS) at room temperature for 20 sec, and once at 50°C for 15 min. Following this stringent wash, strips were rinsed two times with 2 ml of the Innogenetics standard Rinse Solution (RS). Strips were incubated on a rotating platform with the alkaline phosphatase-labelled streptavidin conjugate, diluted in standard Conjugate Solution for 3 min at room temperature. Strips were then washed twice with 2 ml of RS and once with standard Substrate Buffer (SB), and the colour reaction was started by adding BCIP and NBT to the SB. After 30 min at room temperature, the colour reaction was stopped by replacing the colour compounds by distilled water. Immediately after drying, the strips were interpreted. The complete procedure described above can also be replaced by the standard Inno-LiPA automation device (Auto-LiPA, Innogenetics, Zwijnaarde, Belgium).

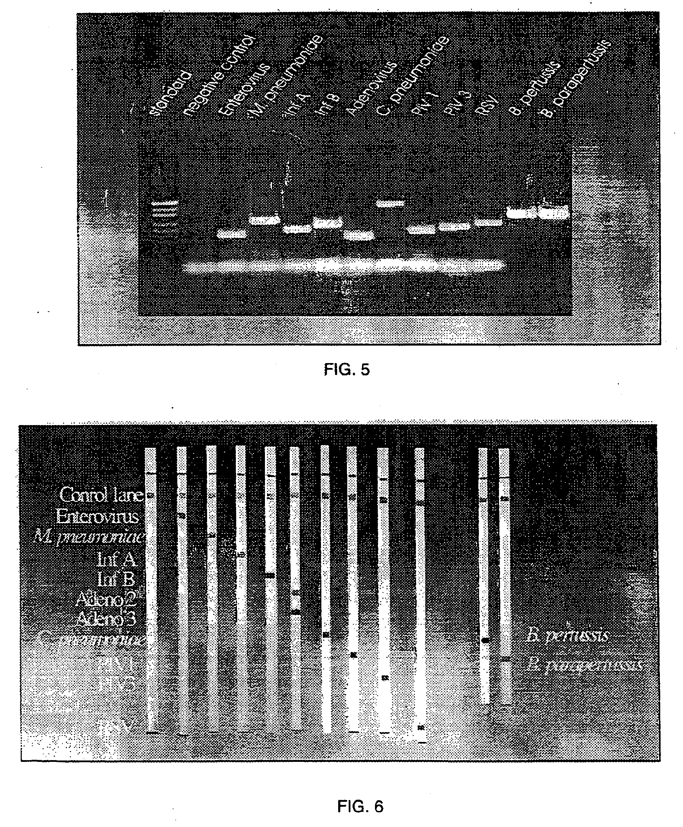

As can be seen in Figure 5, the separation on a 2% agarosegel of the amplicons obtained after multiplex-RT-PCR on reference material shows discrete bands of the expected size for all organisms tested.

Figure 6 shows hybridization results of these amplicons with the LiPA strips as well as a negative control. These results clearly demonstrate that all the probes on the strip react specifically with their corresponding amplicons and no cross-hybridization is seen between the different organisms tested.

### 4. New probe development for C. pneumoniae, M. pneumoniae, B. pertussis and B. parapertussis.

To replace primers and probes (16S rRNA) used in the mulitplex-RT-PCR for detection of *M*. *pneumoniae* and *C. pneumoniae,* a new set of primers and probes was developed for these organisms derived from the 16S-23S rRNA spacer region. Also primers and probes were developed for the specific detection of *B. pertussis* and *B. parapertussis* or *B. bronchiseptica* to add to the multiplex-RT-PCR.

In Table 4, the sequences of the primers and probes, derived from the 16S-23S rRNA spacer region for these bacterial pathogens are summarized.

PCR experiments demonstrated that all selected primersets specifically amplified the corresponding organisms and no amplicons were obtained using nucleic acids derived from phylogenetically related organisms or any of the other infectious agents detected in the multiplex-RT-PCR.

Biotinylated universal primers derived from the 3' end of the 16S rRNA and the 5' part of the 23S rRNA were used to amplify the 16S-23S rRNA spacer region of the bacteria of interest and their closest relatives.

Reverse hybridization on LiPA strips in 2x SSC/0.1% SDS at 50°C showed that all selected probes specifically reacted with the organisms of interest and no cross-reaction was seen with amplicons derived from the previously described multiplex-RT-PCR.

Initial experiments demonstrated that primers and probes (derived from the ribosomal spacer) can be implemented in the multiplex-RT-PCR to replace the currently used primers and probes for *M. pneumoniae* and *C. pneumoniae* and that the assay can be extended by adding primers and probes for Bordetella spp. involved in respiratory tract infections.

From these results it is anticipated that this set can be further extended with probes (more particularly from the 16S-23S rRNA spacer region) for other relevant pathogens such as *Legionella pneumophila.*

### Example 3: m-RT-PCR and LiPA hybridization on culture supernatants

To check the accuracy and specificity of the multiplex-RT-PCR and LiPA hybridization, a number of blinded cultures were analyzed with the LiPA assay.

### 1. Sample preparation and PCR

Nucleic acid preparation was done on culture supernatants using the Boehringer-Mannheim "High Pure Viral Nucleic Acid Kit" as described by the manufacturer. The m-RT-PCR involved the reverse transcription of the RNA from RNA organisms (RSV, PIV1, PIV3, InfA, InfB, enterovirus) followed by a PCR amplification of the corresponding cDNA and the DNA of adenovirus, *M*. *pneumoniae, C. pneumoniae, B. pertussis* and *B. parapertussis* as being described in the previous examples.

Primers were chosen from previously published highly conserved target sequences, except for amplification of the bacterial species, primers used are the following : for *M*. *pneumoniae* SEQ ID NO 17 and SEQ ID NO 19; for *C. pneumoniae* SEQ ID NO 20 and SEQ ID NO 21 and for both Bordetella species SEQ ID NO 22 and SEQ ID NO 23.

### 2. LiPA hybridization

Five to 10 µl of PCR product was hybridized to LiPA strips containing specific probes for the different organisms, as described in Table 3 for enterovirus, influenza A and B, adenovirus and parainfluenzavirus, and in Table 4 for the bacterial species. For RSV, the probes used are as described in Table 5.

Hybridization was done as described in example 2.

### 3. Results

A comparison of culture and LiPA results for a first series of 36 blinded samples is summarized in Table 6.

LiPA results are concordant with culture results in most of the cases. In two cases, multiplex testing revealed the presence of double infections, where culture results only detected one of the two organisms present.

Negative LiPA results obtained were seen with old culture supernatants, possibly due to degradation of nucleic acid material.

In a second experiment, blinded samples for culture of *Mycoplasma pneumoniae* were evaluated using the multiplex-RT-PCR and subsequent LiPA hybridization. Results of 30 blinded specimens are summarized Table 7.

The results in the LiPA testing were 100% concordant to the results obtained in culture.

**Table 1: Comparison of EIA and m-RT-PCR-ELISA**

| ***RSV*** | **EIA** | | | ***InfA*** | **EIA** | | |
|---|---|---|---|---|---|---|---|
| **PCR** | | Pos. | Neg. | **PCR** | | Pos. | Neg. |
| | Pos. | 116 | 25 | | Pos. | 52 | 14 |
| | Neg. | 24 | 866 | | Neg. | 1 | 873 |
| | **Total** | 140 | 891 | | **Total** | 53 | 887 |

**Table 2. Primer sequences used in Example 1**

| | |
|---|---|
| ENTERO- FP1: | att gtc acc ata agc agc ca-3' |
| ENTERO- RP1: | tcc tcc ggc ccc tga atg cg-3' |
| MPN-FP1: | aag gac ctg caa ggg ttc gt-3' |
| MPN-RP1: | ctc tag cca tta cct gct aa-3' |
| INFLUA-FP1: | aag ggc ttt cac cga aga gg-3' |
| INFLUA-RP1: | ccc att ctc att act gct tc-3' |
| INFLUB-FP1: | atg gcc atc gga tcc tca ac-3' |
| INFLUB-RP1: | tgt cag cta tta tgg age tg-3' |
| ADENO-FP1: | gcc gag aag ggc gtg cgc agg ta-3' |
| ADENO-RP1: | atg act ttt gag gtg gat ccc atg ga-3' |
| CPN-FP1: | tga caa ctg tag aaa tac agc-3' |
| CPN-RP1: | cgc ctc tct cct ata aat-3' |
| PIV1-FP1 : | cac atc ctt gag tga tta agt ttg atg a-3' |
| PIV1-RP1: | att tct gga gat gtc ccg tag gag aac-3' |
| PIV3-FP1: | tag cag tat tga agt tgg ca-3' |
| PIV3-RP1: | aga ggt caa tac caa caa cta-3' |
| RSV-FP1: | tgt tat agg cat atc att ga-3' |
| RSV-RP1: | tta acc agc aaa gtg tta ga-3' |

**Table 3.**

| **Organism** | **Original probe** | **Adapted versions*** | **Name**** |
|---|---|---|---|
| | | | |
| Enterovirus | gaaacacggacacccaaagta | gaaacacggacacccaaagta (SEQ ID NO 4) | **entero1** |
| Influenza A | gtcctcatcggaggacttgaatggaatgat | catcggaggacttgaatgg (SEQ ID NO 5) | **influa1** |
| Influenza B | gtcaagagcaccgattatcac | gtcaagagcaccgattatcac (SEQ ID NO 6) | **influb1** |
| Adenovirus | ctcgatgacgccgcggtgc | gatgacgccgcggtg (SEQ ID NO 7) | adeno1 |
| | | tctcgatgacgccgcg (SEQ ID NO 8) | **adeno2** |
| | | cataaagaagggtgggc (SEQ ID NO 9) | **adeno3** |
| Parainfluenza 1 | taccttcattatcaattggtaagtcaatatatg | ccttcattatcaattggtaagtc (SEQ ID NO 10) | piv11 |
| | | ccttcattatcaattggtgatgc (SEQ ID NO 11) | **piv12** |
| | | gttagaytaccttcattatcaattggt (SEQ ID NO 12) | piv13 |
| Parainfluenza 3 | aaaattccaaaagagaccggc | aaaattccaaaagagaccggc (SEQ ID NO 13) | **piv31** |
| RSV | cctgcattaacactaaattc | cacctgcattaacactaaattct (SEQ ID NO 14) | **rsv1** |
| *M. pneumoniae* | actcctacgggaggcagcagta | ctacgggaggcagcagt (SEQ ID NO 15) | **mpn1** |
| *C. pneumoniae* | tcttgctaccttctgtactaa | tcttgctaccttctgtactaa (SEQ ID NO 16) | **cpn1** |

| | | | |
|---|---|---|---|
| *y =c or t ** Probes in bold were used on first generation LiPA assay. | | | |

**Table 4. Sequences of the primers and probes, derived from the 16S-23S rRNA spacer region.**

| **Organism** | **Primers*** | **Probes** |
|---|---|---|
| Mycoplasma pneumoniae | FP1: ggtggatcacctcctttctaatg (SEQ ID NO 17) | MPN3: ggtaaattaaacccaaatccct (SEQ ID NO 24) |
| | FP2 : gtggtaaattaaacccaaatccc (SEQ ID NO 18) | MPN4 : gaacatttccgcttctttc (SEQ ID NO 25) |
| | RP : gcatccaccataagcccttag (SEQ ID NO 19) | MPN5: gaacatttccgcttctttcaa (SEQ ID NO 26) |
| Chlamydia pneumoniae | FP : cctttttaaggacaaggaaggttg (SEQ ID NO 20) | CPN2 : gcaagtattttatattccgcatt (SEQ ID NO 27) |
| | RP : gatccatgcaagttaacttcacc (SEQ ID NO 21) | CPN3 : gttttcaaaacattcagtatatgatc (SEQ ID NO 28) |
| Bordetella pertussis | FP : tatagctgctggatcggtgg (SEQ ID NO 22) | BP2 : gcctgtccagaggatg (SEQ ID NO 29) |
| | RP : ccaaaacccaacgcttaacac (SEQ ID NO 23) | BPP1 : cccgtcttgaagatggg (SEQ ID NO 30) |
| Bordetella | idem B. pertussis | |
| parapertussis/bronchiseptica | | |

| | | |
|---|---|---|
| * : FP = forward primer; RP = reverse primer. | | |

**Table 5. Sequences of probes for RSV, used in example 3.**

| **Probe*** | **Name** |
|---|---|
| tta aca trt aag tgc tta mag (SEQ ID NO 31) | rsv2 |
| cct gca ttr aca cta aat tc (SEQ ID NO 32) | rsv6 |
| cac ctg cat tra cac taa att c (SEQ ID NO 33) | rsv7 |
| ctt aca cct gca ttr aca cta aat tc (SEQ ID NO 34) | rsv8 |

| | |
|---|---|
| * r = a or g, and m = a or c | |

**Table 6. Culture and LiPA results for a series of 36 blinded samples**

| **Specimen** | **Culture-result** | **LiPA-result** |
|---|---|---|
| 1 | Adenovirus | Adenovirus |
| 2 | Adenovirus 4 | Adenovirus |
| 3 | Adenovirus 4 | Adenovirus |
| 4 | Adenovirus | Enterovirus + Adenovirus |
| 5 | Adenovirus | Adenovirus |
| 6 | Echo Type 24 12374/97 | Enterovirus |
| 7 | Echo Type 30 7682/97 | Enterovirus |
| 8 | Inf A/WSN (H1N1) HA:1:1024 | Influenza A |
| 9 | Inf A/Hongkong HA:1:128 | Influenza A |
| 10 | Inf A/Shope/54 HA:1:256 | Influenza A |
| 11 | Inf B/Hongkong HA:1:64 | Influenza A + Influenza B |
| 12 | Inf B/Lee/40 HA:1:64 | Influenza B |
| 13 | Inf B/Bejing/6 HA:1:64 | Influenza B |
| 14 | Inf B/Victoria HA:1:64 | Influenza B |
| 15 | Inf A/Wuhan/371/95 HA:1:32 | Influenza A |
| 16 | Inf AlTexas/36/91 HA:1:256 | Influenza A |
| 17 | Inf A/JHB/33/94 HA:1:256 | negative |
| 18 | Inf B/Harbin/7/94 HA:1:32 | Influenza B |
| 19 | Inf A/Nanchang/93/95 HA:1:64 | Influenza A |
| 20. | Inf B/Singapour/6/86 HA:1:256 | Influenza B |
| 21 | PIV 2 | negative |
| 22 | PIV 2 | negative |
| 23 | PIV 2 | negative |
| 24 | serological Inf A positive | negative |
| 25 | serological Inf A positive | negative |
| 26 | Coxsackie Type B1 | Enterovirus |
| 27 | Coxsackie Type B2 | Enterovirus |
| 28 | Coxsackie Type B3 | Enterovirus |
| 29 | Coxsackie Type B4 | Enterovirus |
| 30 | Coxsackie Type B5 | Enterovirus |
| 31 | Coxsackie Type B6 | Enterovirus |
| 32 | Coxsackie Type A16 | Enterovirus |
| 33 | Echo Type 6 | Enterovirus |
| 34 | Echo Type 7 | Enterovirus |
| 35 | Echo Type 11 | Enterovirus |
| 36 | Echo Type 30 7682/97 | Enterovirus |

**Table 7. Results of 30 blinded specimens for culture of Mycoplasma pneumoniae.**

| ***M. pneumoniae*** | **LiPA positive** | **LiPA negative** |
|---|---|---|
| Culture positive | 17 | 0 |
| Culture negative | 0 | 13 |

### REFERENCES

Adcock, P.M., G.G. Stout, M. A. Hauck, and G.S. Marshall (1997). Effect of rapid viral diagnosis on the management of children hospitalized with lower respiratory tract infection. J. Pediatric. Infect. Dis. 16: 842-846.

Asseline U., Delarue M., Lancelot G., Toulme F. and Thuong N. (1984) Nucleic acid-binding molecules with high affinity and base sequence specificity : intercalating agents covalently linked to oligodeoxynucleotides. Proc. Natl. Acad. Sci. USA 81 (11):3297-301:

Bartlett, J.G., and L.M. Mundy (1995). Community-acquired pneumonia, NEJM, 333: 1618 - 1624.

Bej A., Mahbubani M., Miller R., Di Cesare J., Haff L., Atlas R. (1990) Multiplex PCR amplification and immobilized capture probes for detection of bacterial pathogens and indicators in water. Mol Cell Probes 4:353-365.

Claas, E.C.J., M.J.W. Sprenger, G.E.M. Kleter, R. van Beek, W.G.V. Quint, and N. Masurel (1992). Type-specific identification of influenza viruses A, B and C by the polymerise chain reaction. J. Virol. Meth. 39: 1 - 13.

Compton J. (1991) Nucleic acid sequence-based amplification. Nature 350: 91-92.

Dixon, R.E. (1985). Economic costs of respiratory infections in the United States. Am. J. Med. 78 (Suppl. 6B) : 45 - 51.

Drews, A. L., R. L. Atmar, W.P. Glezen, B.D. Baxter, P.A. Piedra, S.B. Greenberg (1997). Dual respiratory virus infections. Clin. Infect. Dis. 25: 1421 - 1429.

Duck P. (1990) Probe amplifier system based on chimeric cycling oligonucleotides. Biotechniques 9: 142-147.

Echeviarra, J.E., D.D. Erdman, E.M. Swierkosz, B.P. Holloway, L.J. Anderson (1998). Simultaneous detection and identification of human parainfluenza viruses 1, 2, and 3 from clinical samples by multiplex PCR. J. Clin. Microbiol. 36: 1388.- 1391.

Ellis, J.S., D.M. Fleming and M.C. Zambon (1997). Multiplex reverse transcription-PCR for surveillance of influenza A and B viruses in England and Wales in 1995 and 1996. J. Clin. Microbiol. 35: 2076 - 2082.

Falck G., J. Gnarpe, and H. Gnarpe (1997). Prevalence of Chlamydia pneumoniae in healthy children and in children with respiratory tract infections. Pediatr. Infect. Dis. J. 16: 549 - 554.

Fan, J. And K. Henrickson (1996). Rapid diagnosis of human parainfluenza virus type 1 infection by quantitative reverse transcription-CPR-enzyme hybridization assay. J. Clin. Microbiol. 34 : 1914 - 1917.

Garenne, M., C. Ronsmans, H. Campbell (1992). The magnitude of mortality from acute respiratory infections in children under 5 years in developing countries. World Health Stat. Q. 45: 180 - 191.

Gaydos, C.A., T.C. Quin, and J.J. Eiden (1992). Identification of Chlemydia pneumoniae by DNA amplification of the 16S rRNA gene. J. Clin. Microbiol. 30: 796 - 800.

Gendrel, D., J. Raymond, F. Moulin, J.L. Iniguez, S. Ravilly, F. Habib, P. Lebon, and G. Kalifa (1997). Etiology and response to antibiotic therapy of community-acquired pneumonia in French children. Eur. J. Clin. Microbiol. Infect. Dis. 16: 388 - 391.

Gilbert, L.L., A. Dakhama, B.M. Bone, E.E. Thomas, and R.G. Hegele (1996). Diagnosis of viral respiratory tract infections in children by using a reverse transcription-PCR panel, J. Clin. Microbiol. 34: 140 - 143.

Goo, Y.A.M.K. Hori, J.H. Jr. Voorhies, C.C. Kuo, S.P. Wang, L.A. Campbell (1995). Failure to detect Chlamydia pneumoniae in ear fluids from children with otitis media. Pedriatr. Infect. Dis. J. 14: 1000 - 1001.

Guatelli J., Whitfield K., Kwoh D., Barringer K., Richman D. and Gengeras T. (1990) Isothermal, in vitro amplification of nucleic acids by a multienzyme reaction modeled after retroviral replication. Proc. Natl. Acad. Sci. USA 87: 1874-1878.

Hassan-King, M., I. Baldeh, R. Adegbola, C. Omosigho, S.O. Usen, A. Oparaugo, B.M. Greenwood (1996). Detection of Haemophilus influenzae and Streptococcus pneumoniae DNA in blood culture by a single PCR assay. J. Clin. Microbiol. 34: 2030 - 2032.

Hassan-King, M., R. Adegbola, I. Baldeh, K. Mulholland, C. Omosigho, A. Oparaugo, S. Usen, A. Palmer, G. Schneider, O. Secka, M. Weber, B. Greenwood (1998). A polymerase chain reaction for the diagnosis of Heamoplihus influenzae type b disease in children and its evaluation during a vaccine trail. Pediatr. Infect. Dis. J. 17: 309 - 312.

Hemming, V. G. (1994). Viral respiratory diseases in children : classification etiology, epidemiology and risk factors. J. Pediatr. 124: S 13 - 16.

Hierholzer, J.C., P.E. Halonen, P.O. Dahlen, P. G Bingham, M.M. McDonough (1993). Detection of adenovirus in clinical specimens by polymerase chain reaction and liquid-phase hybridization quantitated by time-resolved fluoremetry, J. Clin. Microbiol. 31: 1886 - 1891.

Hinman, A.R. (1998). Global progress in infectious disaeses control. Vaccine 16: 1116 - 1121.

Karron, R.A., K.L. O'Brien, J.L. Froehlich, and V.A. Brown (1992). Molecular epidemiology of a parainfluenza type 3 virus outbreak on a pediatric ward. J. Inf. Dis. 167: 1441 - 1445.

Kawasaki, E.S. (1990). Amplification of RNA. P 21-27 In M.A. Innis, D.H. Gelfand, J.J. Sninsky and T.J. White (ed.), PCR protocols : A guide to methods and applications. Academic Press.

Kwoh D., Davis G., Whitfield K., Chappelle H., Dimichele L. and Gingeras T. (1989). Transcription-based amplification system and detection of amplified human immunodeficiency virus type 1 with a bead-based sandwich hybridization format. Proc Natl Acad Sci USA 86: 1173-1177.

Kwok S., Kellogg D., McKinney N., Spasic D., Goda L., Levenson C. and Sinisky J. (1990) Effects of primer-template mismatches on the polymerase chain reaction: Human immunodeficiency virus type 1 model studies. Nucl. Acids Res. 18: 999.

Landgren U., Kaiser R., Sanders J. and Hood L. (1988) A ligase-mediated gene detection technique. Science 241:1077-1080.

Lockhart, D.J., Dong, H., Byme, M.C., Follettie, M., Gallo, M.V., Chee, M.S., Mitteman, M., Wang, C., Kobayashi, M., Horton, H. and Bown, E.L. (1996). Expression monitoring by hybridisation to high density oligonucleotide arrays. Nature Biotechnology 14: 1675-1680.

Lomeli H., Tyagi S., Pritchard C., Lisardi P. and Kramer F. (1989) Quantitative assays based on the use of replicatable hybridization probes. Clin Chem 35: 1826-1831.

Marx, A., T.J. Török, R.C. Holman, M.J. Clarke, and L.J. Anderson (1997). Pediatric Hospitalizations for croup (laryngotracheobronchitis): biennial increases associated with human parainfluenzavirus 1 epidemics. J. Infect. Dis. 176 : 1423 - 1427.

Matsukura M., Shinozuka K., Zon G., Mitsuya H., Reitz M., Cohen J. and Broder S (1987). Phosphorothioate analogs of oligodeoxynucleotides : inhibitors of replication and cytopathic effects of human immunodeficiency virus. Proc. Natl. Acad. Sci. USA 84 (21):7706-10.

Messmer, T.O., K. Skelton, J. F. Moroney, H. Daugharty, and B.S. Fields (1997). Application of a nested, multiplex PCR to psittacosis outbreaks. J. Clin. Microbiol. 35: 2043 - 2046.

Miller P, Yano J, Yano E, Carroll C, Jayaram K, Ts'o P (1979) Nonionic nucleic acid analogues. Synthesis and characterization of dideoxyribonucleoside methylphosphonates. Biochemistry 18 (23): 5134-43.

Mullis K., Faloona F., Scharf S. et al. Specific enzymatic amplification of DNA in vitro: the polymerase chain reaction (1986). Gold Spring Harb. Symp. Quant. Biol. 1: 263-273.

Mullis K.B. and Faloona F.A. Specific synthesis of DNA in vitro via a polymerase-catalyzed chain reaction (1987). Methods Enzymol. 155: 335-350.

Nielsen P., Egholm M., Berg R. and Buchardt O. (1991) Sequence-selective recognition of DNA by strand displacement with a thymine-substituted polyamide. Science 254 (5037): 1497-500.

Nielsen P., Egholm M., Berg R. and Buchardt O. (1993) Sequence specific inhibition of DNA restriction enzyme cleavage by PNA. Nucl. Acids Res. 21(2): 197-200.

Nohynek, H., J. Eskola, M. Kleemola, E. Jalonen, P. Saikku, M. Leinonen (1995). Bacterial antibody assays in the diagnosis of acute lower respiratory tract infection in children. Pediatr. Infect. Dis. J. 14: 478 - 484.

Paton, A.W., J.C. Paton, A.J. Lawrence, P.N. Goldwater, and R.J. Harris (1992). Rapid detection of respiratory syncytial virus in nasopharyngeal aspirates by reverse transcription and polymerase chain reaction amplification. J. Clin. Microbiol. 30: 901 - 904.

Reznikov, M., T.K. Blackmore, J.J. Finlay-Jones and D.L. Gordon (1995). Comparison of nasopharyngeal and throat swab specimens in a polymerase chain reaction-based test for Mycoplasma pneumoniae. Eur. J. Clin. Microbiol. Infect. Dis.14:58 -61.

Rotbart, H.A. (1990). Enzymatic RNA amplification of the enteroviruses. J. Clin. Microbiol. 28 : 438 - 442.

Rotbart, H.A., M.H. Sawyer, S. Fast, C. Lewinski, N. Murphy, E.F. Keyser, J. Spadoro, S.Y. Kao, M. Loeffelholz (1994). Diagnosis of enteroviral meningitis by using PCR with a colorimetric microwell detection assay. J. Clin. Microbiol. 32: 2590 - 2592.

Saiki R.K., Bugawan T.L., Hom G.T. et al. Analysis of enzymatically amplified betaglobin and HLA-DQ alpha DNA with allele-specific oligonucleotide probes (1986). Nature 324: 163-166.

Saiki, R.K., D.H. Gelfand, S. Stoffel, S.J. Scharf, R. Higuchi, G.T. Hom, K.B. Mullis, H.A. Erlich (1988). Primer-directed enzymatic amplification of DNA with a thermostable DNA polymerase. Science 239: 487 - 491.

Saiki R.K., Walsh P.S., Levenson, C.H. and Erlich H.A. Genetic analysis of amplified DNA with immobilizes sequence-specific oligonucleotide probes (1989). Proc. Natl. Acad. Sci. USA 86: 6230-6234.

Saiki, R.K. (1990). Amplification of genomic DNA, p 13-20. In M.A. Innis, D.H. Gelfand, J.J. Sninsky and T.J. White (ed.) PCR protocols : A guide to methods and applications. Academic Press.

Saikku, P. (1997). Atypical respiratory pathogens. Clin. Microbiol. Inf. 3: 599 - 604.

Sambrook, J., E.F. Fritsch, And T. Maniatis (1989). Molecular cloning: A Laboratory Manual. Cold Spring Harbor Laboratory. Cold Spring Harbor, NY.

Stauffer, F., H. Haber, A. Rieger, R. Mutschlechner, P. Hasenberger, V.J. Tevere, K.K. Young (1998). Genus level identification of mycobacteria from clinical specimens by using an easy-to-handle Mycobacterium-specific PCR Assay. J. Clin. Microbiol. 36: 614 - 617.

Stuyver L., Rossau R., Wyseur A .et al (1993) Typing of hepatitis C virus isolates and characterization of new subtypes using a line probe assay. J. Gen. Virol. 74: 1093-1102

Trolfors, B. And B.A. Claesson (1997). Childhood pneumonia : possibilities for aetiological diagnosis. Baillière's Clinical Paediatrics. 5: 71 - 84.

UNICEF. (1993). Pneumonia, 3.5 millions deaths, The State of the World's Children.

Valassina, M. A.M. Cuppone, M.G. Cusi, P.E. Valensin (1997). Rapid detection of different RNA respiratory virus species by multiplex RT-PCR: application to clinical specimens. Clin. Diagn. Virol. 8: 227 - 232.

Van Kuppeveld, F.J.M. van der Logt, A.F. Angulo, M.J. Van Zoest, W.G.V. Quint, H.G.M. Niesters, J.M.D. Galama, and W.J.G. Melchers (1992). Genus- and species-specific identification of mycoplasmas by 16S rRNA amplification. Appl. Env. Microbiol. 58: 2606-2615.

Woo, P.C., S.S. Chlu, W. H. Seto, M. Pelris (1997). Cost-effectiveness of rapid diagnosis of viral respiratory tract infections in pediatric patients. J. Clin. Microbiol. 35: 1579 - 1581.

W u D. and Wallace B. (1989) The ligation amplification reaction (LAR) - amplification of specific DNA sequences using sequential rounds of template-dependent ligation. Genomics 4:560-569.

Yang S.Y. A standardised method for detection of HLA-A and HLA-B alleles by one-dimensional isoelectric focusing (IEF) gel electrophoresis. Immunobiology of HLA. Histocompatibility testing 1987 (ed. B. Dupont). Springer-Verlag, New York, pp. 332-335.

## Claims

1. Method for the detection of acute respiratory tract infection comprising the simultaneous amplification of several target nucleotide sequences present in a biological sample by means of a primer mixture comprising at least one primer set from each one of the following gene regions :
- the F1 subunit of the fusion glycoprotein gene for RSV,
- the hemagglutininneuraminidase gene for PIV-1,
- the 5' non-coding region of the PIV-3 fusion protein gene,
- 16S rRNA sequence for *M. pneumoniae,*
- 16S rRNA sequence for *C*. *pneumoniae,*
- the 5' non-coding region for enterovirus,
- the non-structural protein gene from influenza A,
- the non-structural protein gene from influenza B, and,
- the hexon gene for adenoviruses.

2. Method according to claim 1 wherein said 16S rRNA primers are replaced by primers from the spacer region between the 16S and the 23S rRNA sequences.

3. Method according to claim 1 or 2 wherein in addition also at least one primer pair for the specific detection of *B. pertussis* and *B. parapertussis* are used.

4. Method according to any of claims 1 to 3 wherein said primers are chosen from Table 2 or Table 4.

5. Method according to any of claims 1 to 4 wherein said amplified products are subsequently detected using a probe, with said probe being preferably selected from Table 3, Table 4 or Table 5.

6. Primer mixture containing at least nine primer sets chosen from the group consisting of SEQ ID NOs 17, 18, 19, 20, 21, 22, 23 and primer sequences of Table 2, whereby the mixture contains at least one primer set from each one of the gene regions listed in claim 1.

7. Probe mixture containing at least nine probes chosen from SEQ ID NOs 5, 6, 7, 8, 9, 10, 11, 12, 13, 14, 15, 16, 24, 25, 26, 27, 28, 29, 30, 31, 32, 33 and 34 and their complementary form and their RNA form wherein T is replaced by U, whereby the mixture contains for each one of the gene regions listed in claim 1 at least one specific probe, for the simultaneous detection of respiratory pathogens.

8. Kit for the detection of acute respiratory tract infection comprising a primer mixture as identified in claim 6 for performing a method according to any one of claims 1-4.

9. Kit for the detection of acute respiratory tract infection comprising a probe mixture as identified in claim 7 for performing a method according to claim 5.

10. Kit according to claim 9, wherein said probes are applied as parallel lines on a solid support, preferably on a nylon membrane.

## Patentansprüche

1. Verfahren zum Nachweis einer akuten Infektion der Atemwege, bei dem man gleichzeitig mehrere in einer biologischen Probe vorhandene Zielnukleotidsequenzen mittels eines Primergemischs amplifiziert, das wenigstens einen Primersatz aus jedem der folgenden Genbereiche umfaßt:
- die F1-Untereinheit des Fusionsglykoprotein-Gens für RSV,
- das Hämagglutininneuraminidase-Gen für PIV-1,
- den 5'-nichtcodierenden Bereich des PIV-3-Fusionsprotein-Gens,
- 16S-rRNA-Sequenz für *M. pneumoniae,*
- 16S-rRNA-Sequenz für *C*. *pneumoniae,*
- den 5'-nichtcodierenden Bereich für Enterovirus,
- das Nicht-Strukturprotein-Gen aus Influenza A,
- das Nicht-Strukturprotein-Gen aus Influenza B und
- das Hexon-Gen für Adenoviren.

2. Verfahren nach Anspruch 1, wobei die 16S-rRNA-Primer durch Primer aus dem Spacer-Bereich zwischen der 16S- und der 23S-rRNA-Sequenz ersetzt werden.

3. Verfahren nach Anspruch 1 oder 2, wobei zusätzlich auch wenigstens ein Primerpaar für den spezifischen Nachweis von *B. pertussis* und *B. parapertussis* verwendet wird.

4. Verfahren nach einem der Ansprüche 1 bis 3, wobei die Primer aus Tabelle 2 oder Tabelle 4 gewählt werden.

5. Verfahren nach einem der Ansprüche 1 bis 4, wobei die amplifizierten Produkte anschließend mit einer Sonde nachgewiesen werden, wobei die Sonde vorzugsweise aus Tabelle 3, Tabelle 4 oder Tabelle 5 ausgewählt wird.

6. Primergemisch mit wenigstens neun Primersätzen, gewählt aus der Gruppe SEQ ID NO 17, 18, 19, 20, 21, 22, 23 und Primersequenzen in Tabelle 2, womit das Gemisch wenigstens einen Primersatz aus jedem der in Anspruch 1 aufgeführten Genbereiche enthält.

7. Sondengemisch mit wenigstens neun Sonden, gewählt aus SEQ ID NO 5, 6, 7, 8, 9, 10, 11, 12, 13, 14, 15, 16, 24, 25, 26, 27, 28, 29, 30, 31, 32, 33 und 34 sowie ihrer komplementären Form und ihrer RNA-Form, wobei T durch U ersetzt ist, womit das Gemisch für jeden der in Anspruch 1 aufgeführten Genbereiche wenigstens eine spezifische Sonde enthält, für den gleichzeitigen Nachweis von Krankheitserregern der Atemwege.

8. Kit für den Nachweis einer akuten Infektion der Atemwege, umfassend ein Primergemisch mit der in Anspruch 6 angegebenen Bedeutung zur Durchführung eines Verfahrens nach einem der Ansprüche 1-4.

9. Kit für den Nachweis einer akuten Infektion der Atemwege, umfassend ein Sondengemisch mit der in Anspruch 7 angegebenen Bedeutung zur Durchführung eines Verfahrens nach Anspruch 5.

10. Kit nach Anspruch 9, wobei die Sonden in parallelen Reihen auf einem festen Träger, vorzugsweise auf einer Nylonmembran, aufgetragen werden.

## Revendications

1. Procédé pour la détection d'une infection aiguë des voies respiratoires comprenant l'amplification simultanée de plusieurs séquences nucléotidiques cibles présentes dans un échantillon biologique au moyen d'un mélange d'amorces comprenant au moins un jeu d'amorces de chacune des régions de gène suivantes :
- la sous-unité F1 du gène de glycoprotéine de fusion pour RSV,
- le gène de l'hémagglutinine neuraminidinase pour PIV-1,
- la région non codante en 5' du gène de la protéine de fusion de PIV-3,
- la séquence d'ARNr 16S pour *M. pneumoniae,*
- la séquence d'ARNr 16S pour *C*. *pneumoniae,*
- la région non codante en 5' d'entérovirus,
- le gène de protéine non structurale d'influenza A,
- le gène de protéine non structurale d'influenza B, et
- le gène d'hexon pour des adénovirus.

2. Procédé selon la revendication 1, dans lequel lesdites amorces d'ARNr 16S sont remplacées par des amorces de la région d'espaceur entre les séquences d'ARNr 16S et 23S.

3. Procédé selon la revendication 1 ou 2, dans lequel, en outre, au moins une paire d'amorces pour la détection spécifique de *B*. *pertussis* et *B. parapertussis* est également utilisée.

4. Procédé selon l'une quelconque des revendications 1 à 3, dans lequel lesdites amorces sont choisies dans le Tableau 2 ou le Tableau 4.

5. Procédé selon l'une quelconque des revendications 1 à 4, dans lequel lesdits produits amplifiés sont ultérieurement détectés en utilisant une sonde, ladite sonde étant de préférence choisie dans le Tableau 3, le Tableau 4 ou le Tableau 5.

6. Mélange d'amorces contenant au moins neuf jeux d'amorces choisis dans le groupe constitué de SEQ ID N° 17, 18, 19, 20, 21, 22, 23 et les séquences d'amorce du Tableau 2, de telle manière que le mélange contienne au moins un jeu d'amorces de chacune des régions de gène listées dans la revendication 1.

7. Mélange de sondes contenant au moins neuf sondes choisies parmi SEQ ID N° 5, 6, 7, 8, 9, 10, 11, 12, 13, 14, 15, 16, 24, 25, 26, 27, 28, 29, 30, 31, 32, 33 et 34 et leur forme complémentaire et leur forme d'ARN où T est remplacé par U, de telle manière que le mélange contienne pour chacune des régions de gène listées dans la revendication 1 au moins une sonde spécifique, pour la détection simultanée de pathogènes respiratoires.

8. Kit pour la détection d'une infection aiguë des voies respiratoires comprenant un mélange d'amorces comme décrit dans la revendication 6 pour mettre en oeuvre un procédé selon l'une quelconque des revendications 1 à 4.

9. Kit pour la détection d'une infection aiguë des voies respiratoires comprenant un mélange de sondes comme décrit dans la revendication 7 pour mettre en oeuvre un procédé selon la revendication 5.

10. Kit selon la revendication 9, dans lequel lesdites sondes sont appliquées sous forme de lignes parallèles sur un support solide, de préférence sur une membrane en nylon.
